# EUROPÄISCHE PATENTSCHRIFT

(11) **EP 1 599 710 B1**
(45) Veröffentlichungstag und Bekanntmachung des Hinweises auf die Patenterteilung: **17.11.2010**
(21) Anmeldenummer: 03750621.9
(22) Anmeldetag: 25.09.2003
(51) Int. Cl.: G01L 5/00, G01L 5/16, A61B 5/00

(54) **BEFESTIGUNGSMITTEL MIT TELEMETRISCHER MESSUNG PHYSIKALISCHER GRÖSSEN**
FASTENER PROVIDED WITH A TELEMETRIC SYSTEM FOR MEASURING PHYSICAL QUANTITIES
ELEMENT DE FIXATION COMPORTANT UN SYSTEME DE MESURE TELEMETRIQUE DE GRANDEURS PHYSIQUES

(30) Priorität: 04.03.2003 EP 03004696
(43) Veröffentlichungstag der Anmeldung: 30.11.2005
(73) Patentinhaber: ETH Zurich, 8092 Zürich (CH)
(72) Erfinder: LANG, Udo, CH-8057 Zürich (CH); HIEROLD, Christofer, CH-5400 Baden (CH)
(74) Vertreter: Kley, Hansjörg
(86) Internationale Anmeldenummer: PCT/EP2003/010650
(87) Internationale Veröffentlichungsnummer: WO 2004/079317

(56) Entgegenhaltungen:
- WO-A-00/56210
- WO-A-01//12092
- DE-A- 19 726 830
- US-A- 4 485 681
- US-A- 5 328 551
- US-A- 5 328 551
- DATABASE WPI Week 199337 Derwent Publications Ltd., London, GB; AN 1993-294508 XP002267946 RADCHENKO, YURI: "strain-gauge force sensor" -& SU 1 763 910 A (TSNII MASH), 23. September 1993 (1993-09-23)
- CLAES W ET AL: "A low power miniaturized autonomous data logger for dental implants" SENSORS AND ACTUATORS A, ELSEVIER SEQUOIA S.A., LAUSANNE, CH, Bd. 97-98, 1. April 2002 (2002-04-01), Seiten 548-556, XP004361650 ISSN: 0924-4247

## Beschreibung

Die vorliegende Erfindung betrifft ein Befestigungsmittel mit telemetrischer Messung physikalischer Grössen nach dem Oberbegriff des Patentanspruchs 1.

Die vorliegende Erfindung befasst sich mit der Messung einer physikalischen Grösse an einer Verbindungsstelle zweier Objekte, die durch ein Befestigungsmittel zusammengehalten werden.

Dazu ist in der Schrift US 5,138,885 [5] eine Schraube offenbart, die einen piezoelektrischen Sensor enthält, um auftretende Drücke an der Verbindungsstelle mit einer hohen Genauigkeit messen zu können.

Insbesondere auf dem Gebiet der Chirurgie ist es wünschenswert, für die Verfolgung des Heilungsprozesses Messwerte zu erhalten. In den Schriften [3] und [4] wird dazu ein miniaturisiertes Element und eine zugehörige Auswerteschaltung vorgeschlagen, das für die Fixation von Zahnprothesen mit zusätzlichen Befestigungsmitteln vorgesehen ist und das eine Mehrzahl von Dehnungsmessstreifen enthält. Für die Messung von Kräften werden Dehnungsmessstreifen auf der Mantelfläche einer Hülse aufgebracht. Diese Elemente erfordern einerseits eine Draht- bzw. Kabelverbindung zu einer Auswerteschaltung und separat die gebräuchlichen Befestigungsmittel wie z.B. Implantatschrauben. Es ist offensichtlich, dass eine Verfolgung des Heilungsprozesses oder auch nur das Messen einer physikalischen Grösse am lebenden Körper eine kaum zumutbare Beeinträchtigung des betreffenden Individuums darstellt, sei es nun ein Mensch oder ein Tier.

In WO 2001/12092 A1 [6] ist ein als Implantat ausgebildeter Sensor offenbart, dessen erfasste physikalische Grössen durch eine ausserhalb des Körpers befindliche Abfrageeinheit ausgewertet werden können. Der Sensor ist als LRC-Glied ausgebildet, so dass die Abfrage sich relativ einfach gestaltet.

Der vorliegenden Erfindung liegt daher die Aufgabe zugrunde, ein Befestigungsmittel insbesondere als Implantat anzugeben, das auch beim Einsatz am lebenden Körper keine unzumutbare Beeinträchtigung der Lebensqualität zur Folge hat und besonders einfach in einem chirurgischen Eingriff anzuwenden ist.

Diese Aufgabe wird durch ein Befestigungsmittel mit den im Patentanspruch 1 angegebenen Merkmalen gelöst. Vorteilhafte Ausgestaltungen der Erfindung sind in weiteren Ansprüchen angegeben.

Das vorliegende Befestigungsmittel weist den wichtigen Vorteil auf, dass keine Kabelverbindung mehr zu einer Auswerteeinheit erforderlich ist und lediglich eine oder mehrere der Befestigungsmittel direkt eingesetzt werden können. Dies ist gegeben durch die Kapselung von Elektronik und Sensorik. Eine solche Kapselung ist für die Biokompatibilität sowie für die Robustheit der Konstruktion von grossem Vorteil. In einer besonderen Ausführungsform der vorliegenden Erfindung werden Dehnungsmessstreifen auf der Basis biologisch degradierbarer, leitfähiger Polymere eingesetzt.

Die Erfindung wird nachfolgend anhand der Zeichnung beispielsweise näher erläutert. Dabei zeigt:
- Figur 1: Zylindrischer Körper als Ausgangsobjekt;
- Figur 2: Anordnung der Dehnungsmessstreifen auf einer fle- xiblen Polymerfolie für einen zylindrischen Körper;
- Figur 3: gerollte Polymerfolie;
- Figur 4: Explosivdarstellung der weiteren Komponenten eines Befestigungsmittels;
- Figur 5: Anordnung der Dehnungsmessstreifen auf einer Folie für eine Ausführungsform;
- Figur 6: Darstellung eines Schraubenkopfes im montierten Zustand;
- Figur 7: Anordnung von drei Dehnungsmessstreifen auf einer flexiblen Polymerfolie für einen zylindrischen Körper zur Messung von Biegemomenten.

Ein Befestigungsmittel wird anhand eines zylindrischen Körpers in Form einer Schraube erläutert. Die Herstellung einer solchen Schraube erfolgt gemäss den nachfolgend aufgeführten Verfahrensschritten:
Schritt 1:

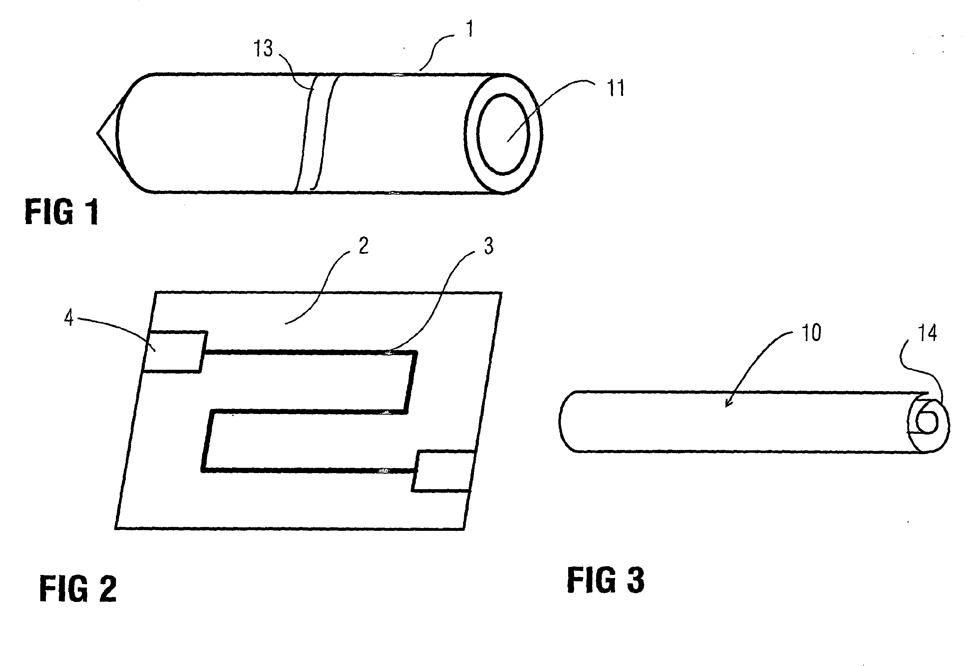

   Fig. 1 zeigt einen zylindrischen Körper 1, der vorzugsweise aus hochfestem, gegebenenfalls biokompatiblem Material besteht. Dem Körper 1 können Gewindegänge 13 durch Walzen oder Drehen bereits aufgebracht sein. Der Körper 1 wird aufgebohrt und es entsteht dadurch ein Innenraum 11.
Schritt 2:
   Gemäss Darstellung in Figur 2 wird auf einer dünnen, flexiblen Polymerfolie 2 durch Mikrostrukturierung ein Dehnungsmessstreifen 3 - abkürzt DMS - hergestellt. Diese Herstellung erfolgt durch Aufdampfen einer Metallschicht durch Schattenmaske, Nassätzen einer aufgesputterten Metallschicht oder
   Strukturierung einer thermoplastischen, leitfähigen Polymerschicht durch Heissprägen einer leitfähigen Schicht. Das Verfahren des Heissprägens ist in [1] beschrieben. Die Strukturierung erfolgt dabei derart, dass zwei grosse Kontaktflächen 4 entstehen, jeweils eine auf beiden Kopfseiten.
   Diese Kontaktflächen 4 können auch um den Rand der Folien 2 ausgeführt sein, so dass sie auf beiden Seiten der Folie 2 liegen. Bei der thermoplastischen, leitfähigen Polymerschicht kann es sich dabei um biologisch degradierbare, leitfähige Polymere handeln, wie sie beispielsweise in [2] offenbart sind.
Schritt 3:
   Anschliessend wird diese Folie 2 zu einer Rolle 14 derart aufgerollt- siehe dazu die Figur 3, dass vorzugsweise die langen Stege des DMS 3 in Längsrichtung zeigen. Die Aussenhaut dieser Rolle 14 wird teilweise oder vollständig mit (leitfähigem) Klebstoff 10 bestrichen und in den Innenraum 11 der Metallhülse 1 eingeführt. Die Kontaktierung des DMS 3 erfolgt somit über eine Kontaktfläche 4 innerhalb der Metallhülse 1 und über die zweite Kontaktfläche 4 und die Metallhülse 1 selbst.
Schritt 4:

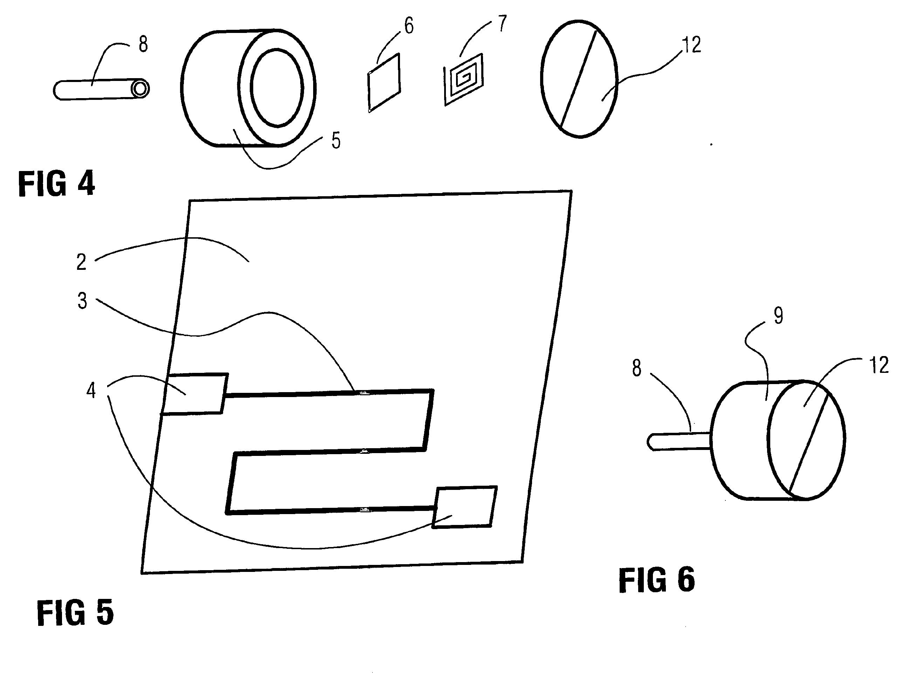

   Figur 4 zeigt eine Explosivdarstellung eines Schraubenkopfes 9. Ein hohlzylinderförmiger Deckel 5 ist so gefertigt, dass er eine integrierte Schaltung 6 sowie eine Antenne 7 zur telemetrischen Auslesung aufnehmen kann. Zusätzlich kann - falls erforderlich - eine Batterie integriert werden. Alternativ könnte auch eine Energieversorgung durch ein elektromagnetisches Wechselfeld erfolgen um dadurch die Funktion einer Sendeeinrichtung 6 zu gewährleisten. Dabei werden vom Sensor 3 detektierte Signale der Sendeeinrichtung 6 zugeführt und anschliessend an eine Aussenstelle übermittelt. Die integrierte Schaltung 6 kann nicht nur zur Messung von Dehnungen genutzt werden, sondern auch um Funktionen zur Messung und Übertragung weiterer allenfalls zusätzlicher physikalischer Daten wie beispielsweise Druck, Temperatur, pH-Wert, Beschleunigung, usw. Dabei sind die entsprechenden Sensoren beispielsweise ebenfalls auf der Folie oder separat im Innenraum 11 der Metallhülse 1 angeordnet. Beim Entwurf ist darauf zu achten, dass Deckel 5 und Stift 8 von einander isoliert sind, da der Stift 8 zur Kontaktierung der inneren Kontaktfläche und der Deckel 5 zur Kontaktierung der Hülse verwendet wird. Das Schlitzteil 12 wird auf den Deckel 5 aufgeklebt. Figur 6 zeigt den Schraubenkopf 9 in montiertem Zustand. Schraubenkopf 9 wird in Fügetechnik mit der Metallhülse 1 verbunden. Dadurch erfolgt auch eine Kontaktierung bei der Endmontage, indem der Schraubkopf 9 auf die Metallhülse 1 geschoben und z.B. auf die vorgenannte Weise befestigt wird.

Eine Ausführungsform gemäß der vorliegenden Erfindung wird anhand der Figur 5 erläutert. In Figur 5 ist eine Folie 2 gezeigt, die im wesentlichen auf die gleiche Weise wie in der ersten Ausführungsform mit einem Dehnungsmessstreifen 3 und Kontaktflächen 4 versehen wird. Unterschiedlich sind die Materialien und die Anordnung. Die Anordnung unterscheidet sich vorteilhafterweise dadurch, dass die Kontaktflächen 4 und der DMS 3 auf der Folie asymmetrisch angeordnet sind. Die Asymmetrie ist so, dass beim Aufrollen der Folie die Kontaktflächen 4 und der DMS 3 in den inneren Windungen der Rolle 14 enthalten sind, so dass die äusseren Windungen nur die Folie allein aufweisen, d.h. ohne aufgebrachte Kontaktflächen 4 und DMS 3. Durch diese Anordnung kann nachträglich auf die Rolle 14 ein Gewinde aufgebracht werden (nicht dargestellt in den Figuren), ohne dass davon die elektrischen Verbindungen im Innern dadurch beeinträchtig werden. Gewindegänge können beispielsweise durch Drehen aufgebracht werden. Als Materialen können gewählt werden:
Folie: Kunststoff oder Metallfolie, vorzugsweise Titanfolie. Ist die Folie 2 metallisch, ist vor der Aufbringung von DMS 3 und Kontaktflächen 4 eine isolierende Schicht aufzutragen. In dieser Ausführungsform der vorliegenden Erfindung können auch wenigstens zwei DMS 3 auf der Folie so aufgebracht und angeordnet werden, dass im gerollten Zustand der Folie auch Biegemomente gemessen werden könnten. Wie in der ersten Ausführungsform kann die Rolle 14 mit einem Schraubenkopf 9 abgeschlossen werden.

Bei den beiden vorgenannten Befestigungsmitteln ist beispielsweise die Anordnung der DMS 3 oder anderer Sensoren frei kombinierbar ebenso ist auch der Abschluss nicht an eine der beiden Befestigungsmittel gebunden. Beim Abschluss des Innenraums 11 ist gegebenenfalls zu berücksichtigen, dass dieser Abschluss für bestimmte physikalische Grössen durchlässig sein muss, beispielsweise für die Messung eines pH-Wertes eines Fluids.

Die beiden in den Figuren 1 bis 6 erläuterten Befestigungsmittel erlauben die Messung von auf das Befestigungsmittel ausgeübten Zug- oder Druckkräften. Nachfolgend wird noch ein drittes Befestigungsmittel erläutert, bei der unter Verwendung mehrerer geeignet angeordneter Dehnungsmessstreifen auch eine Biegebeanspruchung auf das Befestigungsmittel gemessen werden kann und die resultierenden Signale an eine externe Auswerteeinheit übermittelt werden können.

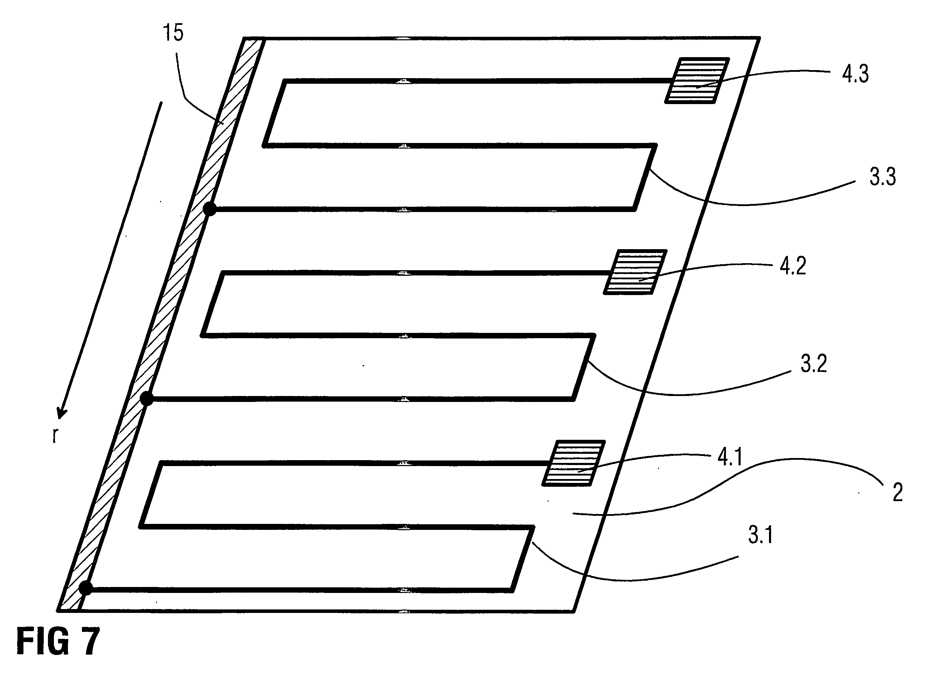

In Figur 7 ist eine Anordnung von drei Dehnungsmessstreifen 3.1, 3.2 und 3.3 auf einer flexiblen Polymerfolie 2 gezeigt. Die Polymerfolie 2 wird dabei wie bei der ersten Ausführungsform in Richtung r gerollt. Auf der Polymerfolie 2 sind im rotatorischen "Abstand" von 120° - nach erfolgtem Aufrollen - drei Dehnungsmessstreifen 3.1, 3.2 und 3.3 mit je einer Kontaktfläche 4.1, 4.2 und 4.3 dargestellt. Die Dehnungsmessstreifen 3.1, 3.2 und 3.3 sind am elektrisch anderen Ende mit Masse 15 verbunden. Masse 15 und die Kontaktflächen 4.1, 4.2 und 4.3 werden vorzugsweise durch Bonden mit der Sendeeinrichtung 6 verbunden. Es ist auch möglich, die Masse mit der Zylinderhülse galvanisch zu verbinden, beispielsweise mit einem leitfähigen Klebstoff. Dabei könnte die Masse 15 auch um den "Rand" geschlagen werden.

In der Sendeeinrichtung 6 wird die Herkunft der von den Dehnungsmessstreifen entstammenden Signale in einem Datenfeld eines Datensatzes codiert, so dass auf einer abgesetzten Auswerteeinheit aus den drei Messergebnissen ein Biegemoment berechnet werden kann. Die drei Sensoren 3.1, 3.2 und 3.3 auf der Polymerfolie 2 können direkt mit dem Stift 8 so kontaktiert werden, dass dieser drei voneinander isolierte um 120° versetzte Finger aufweist. Diese Finger werden durch drei in den Stift gefräste Schlitze gebildet.

Die Messung von Biegemomenten kann auch erreicht werden, in dem drei solche Polymerfolien 2 ineinander bezüglich der Dehnungsmessstreifen um 120° versetzt gerollt werden.

Diese Ausführung zur Messung auftretender Biegemomente kann bezüglich der konstruktiven Ausgestaltung frei kombiniert werden mit den in den Figuren 1 bis 6 beschriebenen Befestigungsmitteln.

Da oft mehrere Befestigungsmittel lokal eingesetzt werden, z.B. für die Verbindung eines gebrochenen Knochens, ist bei der Sendeeinrichtung noch ein weiteres Datenfeld eines Datensatzes vorzusehen, das die betreffende Sendeeinrichtung bzw. das betreffende Befestigungsmittel identifiziert. Damit ist es möglich, aufgrund der übermittelten und ausgewerteten Signale Rückschlüsse auf die betreffende Stelle zu ziehen.

Das Befestigungsmittel 1 ist nicht auf die Anwendung und Verwendung bezüglich Chirurgie beschränkt, sondern kann vorteilhafterweise überall dort angewendet werden, wo einerseits eine kontinuierliche Messung oder eine drahtlose Messung physikalischer Grössen an einer oder um eine Verbindungsstelle erforderlich ist und andererseits keine zusätzlichen Sensoren eingesetzt werden können.

### Liste der verwendeten Bezugszeichen und Abkürzungen

- 1: Zylindrischer Körper; Metallhülse
- 2: Folie, Polymerfolie
- 3: Dehnungsmessstreifen
- 3.1, 3.2, 3.3: Dehnungsmessstreifen
- 4: Kontaktfläche
- 4.1, 4.2, 4.3: Kontaktfläche
- 5: Deckel
- 6: integrierte Schaltung; Sendeeinrichtung
- 7: Antenne
- 8: Stift
- 9: Schraubenkopf, Kopf
- 10: leitfähiger Klebstoff auf gerollter Polymerfolie
- 11: Innenraum
- 12: Schlitzteil
- 13: Gewinde, Gewindegang
- 14: Rolle, gerollte Folie
- 15: Masse

### Liste der verwendeten Abkürzungen

### DMS Dehnungsmessstreifen

### Literaturliste

[1] Annani et al. : «Microfabrication technology for polycaptrolactone, a biodegradable polymer» J Micromech Microeng 10 (2000) 80-84
[2] TyTell et al. : «Synthesis o1 a Novel, Biodegradahle Electrically Conducting Polymer» 107 Biomedical Applications, Adv Funct Mat 12 No 1(2002) 33-37
[3] Puers et al. : «Towards the limits in detecting low-level strain with multiple piezo-resistive sensors» Sensors and Actuators 85 (2000) 395-401
[4] Puers et al. : «A Miniaturized, Autonomous, Programmable Stress Monitoring Device, part of a Dental Prosthesis» Transducers ,01, München.
[5] US 5,138,885
   «Piezoelectric-type pressure sensor» Matsushita Electric Industrial Co. Ltd; Osaka, JP.
[6] WO 2001 / 12092 A1
   «Remotely interrogated diagnostic implant device with electrically passive sensor
   BF Goodrich company; Charlotte; US.

## Patentansprüche

1. Befestigungsmittel (1) enthaltend wenigstens einen Sensor (3) zur Messung einer physikalischen Grösse, wobei
- der Sensor (3) mit einer Sendeeinrichtung (6) verbunden ist, um vom Sensor (3) detektierte Signale zu übermitteln und dass der Sensor (3) und die Sendeeinrichtung (6) im Innenraum (11) des Befestigungsmittels (1) angeordnet sind;
- das Befestigungsmittel (1) aus einer aus einer rechteckigen Folie (2) gebildeten Rolle (14) besteht, wobei
der Sensor (3) und Kontaktflächen (4) asymmetrisch auf der Folie(3) aufgebracht sind, **dadurch gekennzeichnet, dass** die äusseren Windungen der Rolle (14) nur aus der Folie (2) bestehen.

2. Befestigungsmittel (1) nach Anspruch 1,
**dadurch gekennzeichnet, dass**
auf den äusseren Windungen der Rolle (14) ein Gewinde aufgebracht ist.

3. Befestigungsmittel (1) nach Anspruch 1 oder 2,
**dadurch gekennzeichnet, dass**
die Folie (2) aus Titan besteht.

4. Befestigungsmittel (1) nach Anspruch 1 oder 2,
**dadurch gekennzeichnet, dass**
die Folie (2) aus einem biologisch abbaubaren Material besteht.

5. Befestigungsmittel (1) nach einem der Ansprüche 1 bis 4,
**dadurch gekennzeichnet, dass**
drei Sensoren (3.1, 3.2, 3.3) rotatorisch um 120° versetzt angeordnet sind, die die Beanspruchung des Befestigungsmittels (1) durch auftretende Biegemomente erfassen.

6. Befestigungsmittel (1) nach Anspruch 5,
**dadurch gekennzeichnet, dass**
rotatorisch um 120° versetzt zwischen den drei Sensoren (3.1, 3.2, 3.3) Schlitze angeordnet sind.

7. Befestigungsmittel (1) nach Anspruch 6,
**dadurch gekennzeichnet, dass**
die durch die Schlitze gebildeten Finger mit je einem Sensor (3.1, 3.2, 3.3) elektrisch kontaktiert sind und dass die Finger voneinander isoliert sind.

8. Befestigungsmittel (1) nach einem der Ansprüche 1 bis 7,
**dadurch gekennzeichnet, dass**
die Sendeeinrichtung (6) zur Versorgung mit elektrischer Energie mit einer Batterie verbunden ist.

9. Befestigungsmittel (1) nach Anspruch 8,
**dadurch gekennzeichnet, dass**
die Batterie im Innenraum (11) des Befestigungsmittels (1) angeordnet ist.

## Claims

1. Fastener (1) containing at least one sensor (3) for measuring a physical quantity, with
- the sensor (3) being connected to a transmit device (6) in order to transfer signals detected by the sensor (3) and the sensor (3) and the transmit device (6) being arranged in the interior (11) of the fastener (1);
- the fastener (1) consisting of a roller (14) formed from a rectangular foil (2),
with
the sensor (3) and contact surfaces (4) being applied asymmetrically to the foil (3), **characterised in that** the outer windings of the roller (14) only consist of the foil (2).

2. Fastener (1) according to claim 1,
**characterized in that**
a thread is applied to the outer windings of the roller (14).

3. Fastener (1) according to claim 1 or 2,
**characterized in that**
the foil (2) consists of titanium.

4. Fastener (1) according to claim 1 or 2,
**characterized in that**
the foil (2) consists of a biologically degradable material.

5. Fastener (1) according to one of claims 1 to 4,
**characterized in that**
three sensors (3.1, 3.2, 3.3) are arranged rotationally offset by 120°, which detect the strains arising in the fastener (1) from bending moments which occur.

6. Fastener (1) according to claim 5,
**characterized in that**
slots are arranged rotationally offset by 120° between the three sensors (3.1, 3.2, 3.3).

7. Fastener (1) according to claim 6,
**characterized in that**
the fingers formed by the slots are in electrical contact with a sensor (3.1, 3.2, 3.3) in each case and that the fingers are insulated from one another.

8. Fastener (1) according to one of claims 1 to 7,
**characterized in that**
the transmit device (6) is connected to a battery to supply it with electrical energy.

9. Fastener (1) according to claim 8,
**characterized in that**
the battery is arranged in the interior (11) of the fastener (1).

## Revendications

1. Moyen ( 1 ) de fixation comportant au moins un capteur ( 3 ) de mesure d'une grandeur physique, dans lequel
- le capteur ( 3 ) est relié à un dispositif ( 6 ) d'émission, pour transmettre des signaux détectés par le capteur ( 3 ) et en ce que le capteur ( 3 ) et le dispositif ( 6 ) d'émission sont disposés à l'intérieur ( 11 ) du moyen ( 1 ) de fixation ;
- le moyen ( 1 ) de fixation est constitué d'un rouleau ( 14 ) formé d'une feuille ( 2 ) rectangulaire,
dans lequel le capteur ( 3 ) et des surfaces ( 4 ) de contact sont mis dissymétriquement sur la feuille ( 3 ), **caractérisé en ce que** les spires extérieures du rouleau ( 4 ) ne sont constituées que de la feuille ( 2 ).

2. Moyen ( 1 ) de fixation suivant la revendication 1,
**caractérisé en ce que**
un filetage est mis sur les spires extérieures du rouleau ( 14 ).

3. Moyen ( 1 ) de fixation suivant la revendication 1 ou 2,
**caractérisé en ce que**
la feuille ( 2 ) est en titane.

4. Moyen ( 1 ) de fixation suivant la revendication 1 ou 2,
**caractérisé en ce que**
la feuille ( 2 ) est en un matériau biodégradable.

5. Moyen ( 1 ) de fixation suivant l'une des revendications 1 à 4,
**caractérisé en ce que**
trois capteurs ( 3.1, 3.2, 3.3 ) sont décalés en rotation de 120° et relèvent la sollicitation du moyen ( 1 ) de fixation par des couples de flexion qui se produisent.

6. Moyen ( 1 ) de fixation suivant la revendication 5,
**caractérisé en ce que** des fentes sont disposées de manière décalées en rotation de 120° entre les trois capteurs ( 3.1, 3.2, 3.3 ).

7. Moyen ( 1 ) de fixation suivant la revendication 6, **caractérisé en ce que**
les doigts formés par les fentes sont mis en contact électriquement avec respectivement un capteur ( 3.1, 3.2, 3.3 ) et **en ce que** les doigts sont isolés les uns des autres.

8. Moyen ( 1 ) de fixation suivant l'une des revendications 1 à 7,
**caractérisé en ce que**
le dispositif ( 6 ) d'émission est relié à un générateur électrochimique, pour l'alimentation en énergie électrique.

9. Moyen ( 1 ) de fixation suivant la revendication 8,
**caractérisé en ce que**
le générateur électrochimique est disposé à l'intérieur ( 11 ) du moyen ( 1 ) de fixation.
